(19) Europäisches Patentamt
European Patent Office
Office européen des brevets

(11) Publication number: 0 041 760
B1

(12) EUROPEAN PATENT SPECIFICATION

(45) Date of publication of patent specification: 07.03.84

(51) Int. Cl.³: C 07 C 93/06 //A61K31/135

(21) Application number: 81200616.1

(22) Date of filing: 03.06.81

# (54) Process for the preparation of 1-isopropylamino-3-(4-(2-methoxy-ethyl)-phenoxy)-2-propanol.

(30) Priority: 04.06.80 FI 801792
01.10.80 FI 803119

(43) Date of publication of application:
16.12.81 Bulletin 81/50

(45) Publication of the grant of the patent:
07.03.84 Bulletin 84/10

(84) Designated Contracting States:
AT CH IT LI NL SE

(56) References cited:
GB - A - 1 308 106

(73) Proprietor: Aktiebolaget Hässle
Kärragatan 5
S-431 83 Mölndal (SE)

(72) Inventor: Koskenniska, Lasse Antero
Liistetie 4 A 2
SF-90650 Oulu 65 (FI)
Inventor: Eloranta, Maire Marjatta
Puistokatu 24 C 10
SF-90120 Oulu 12 (FI)

(74) Representative: Wurm, Bengt Runio et al,
Patent and Trade Mark Department AB ASTRA
S-151 85 Södertälje (SE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 041 760 B1

Process for the preparation of 1-isopropylamino-3-[4-(2-methoxyethyl)phenoxy]-2-propanol

This invention relates to a new process for the preparation of 1-isopropylamino-3-]4-(2-methoxyethyl)phenoxy]-2-propanol or metoprolol (I) and the acid addition salts thereof.

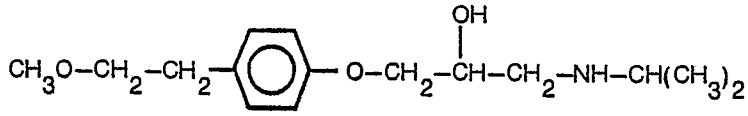

(I)

Metoprolol is a therapeutically valuable so-called β-adrenergic blocker. Most of the β-blockers have the drawback of blocking in addition to the β-receptors of the heart also the β-receptors of the blood vessels and the lungs. Contrary to this, metoprolol is heart specific and thus an important drug in the therapy of heart diseases.

The preparation of metoprolol is known from e.g. the Swedish patent No. 354 851, in which 12 processes for its preparation are described. These processes all relate to methods for the synthesis of the isopropylamino side-chain.

The present invention concerns a process, which in a simple and new way gives highly pure metoprolol. As starting material in the process is used 1,2-epoxy-3-[4-(1-acetoxy-2-methoxyethyl)-phenoxy]-propane (II), which is reacted with isopropylamine to give 1-isopropylamino-3-[4-(1-acetoxy-2-methoxyethyl)phenoxy]-2-propanol (III), which is a new compound. In the following step the product III is reduced either by catalytic hydrogenolysis or with sodium borohydride to metoprolol (I), which then is converted to e.g. its tartrate salt.

The compound 1,2-epoxy-3-[4-(1-acetoxy-2-methoxyethyl)phenoxy]-propane is easily prepared e.g. from 1,2-epoxy-3-[4-(1-hydroxy-2-methoxyethyl)phenoxy]-propane and aceticanhydride or from 4-(1-acetoxy-2-methoxyethyl)phenol or an alkali salt thereof and epichlorohydrine.

The process of the invention can thus be described by the following reaction scheme:

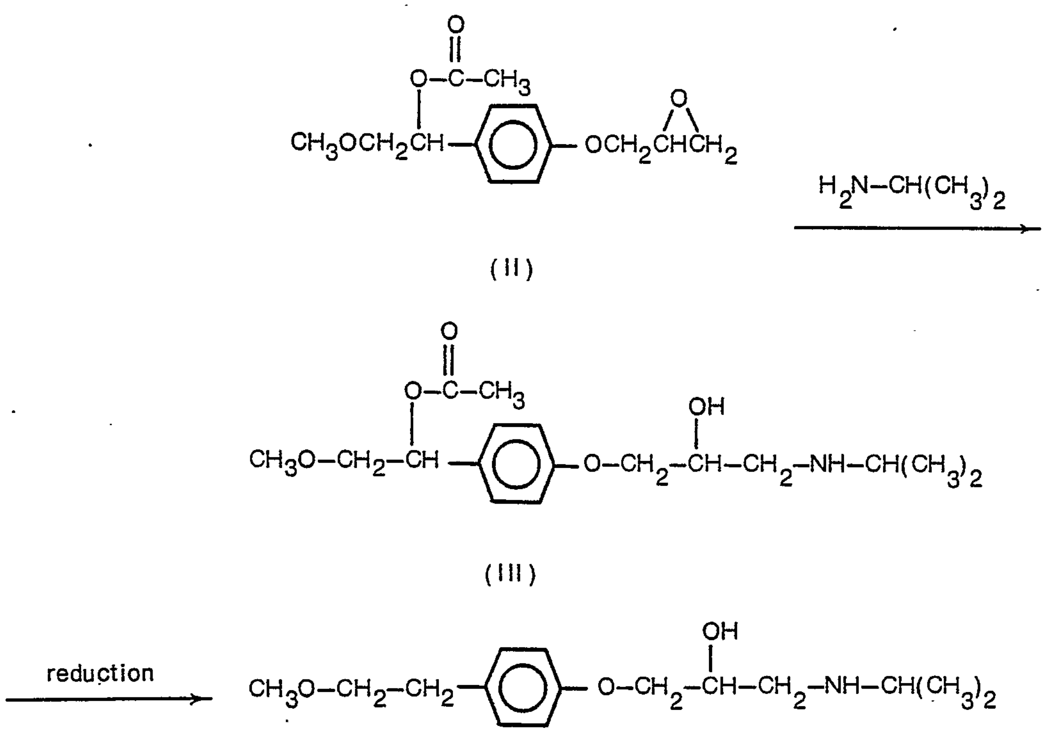


(II)

(III)

(I)

Hydrogenation of the benzyl group is in itself a known reaction (see e.g. Organic Reactions 7 (1953) 263), but never before was an alkylester or arylester group removed from an α-alkylsubstituted alkyl- or arylbenzyl ester by catalytic hydrogenation or reduction with sodium borohydride. The following reaction was thus not known before:

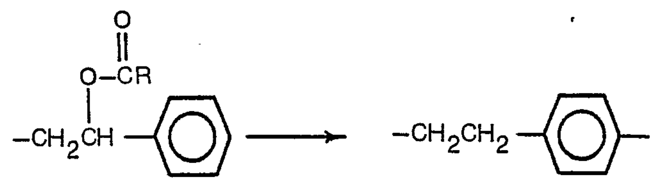

wherein R is an alkyl or aryl group.

The new process of the invention for the preparation of 1-isopropylamino-3-[4-(2-methoxyethyl)phenoxy]-2-propanol or metoprolol (I) thus also provides a new process for removing an alkylester or arylester group from an $\alpha$-alkylsubstituted benzlester by a reduction process.

The above described reaction series is preferably performed in such a way, that the ester-intermediate (III) is isolated. It can be isolated e.g. from an aqueous solution by adding sodium hydrogencarbonate (pH about 7,5) and extracting with an organic solvent. The watersoluble impurities then remain in the aqueous phase. Such impurities are e.g. the reaction products of isopropylamine with epichlorohydrine and other watersoluble impurities.

This isolation of the ester-intermediate is an improvement as compared to our earlier Finnish patent applications 773391, 781001 and 792950. In this application the corresponding intermediates which contain a keto group or an alcohol group, cannot be purified in this way. They are not liberated at pH 7,5 and are, on the other hand, also too watersoluble.

Advantages resulting from the isolation are:

— the reduction is facilitated, since smaller amounts of catalyst are required

— the end product of the reduction, crude metoprolol, is purer and the final purification is easier.

The whole reaction series can of course be performed without isolation of the ester, but isolation of the ester is recommended.

1-Isopropylamino-3-[4-(1-acetoxy-2-methoxyethyl)phenoxy]-2-propanol is prepared by heating the corresponding epoxy compound II with isopropylamine at about 100°C in an autoclave or by refluxing in a suitable solvent, e.g. toluene.

In the final step the benzyl ester III is reduced either by catalytic hydrogenolysis or with sodium borohydride. The catalytic hydrogenation is preferably performed with a palladium-on-carbon catalyst. The reaction proceeds with sufficient speed already at room temperature, and even at 100°C the reaction succeeds. It was very surprising that the reaction occurred so easily at elevated temperature, and even more so at room temperature and normal pressure. The reduction with sodium borohydride is performed in an inert solvent, as for instance methylene chloride, in the presence of trifluoroacetic acid or methanesulfonic acid at a low temperature. At last the product is converted to e.g. the tartrate salt.

The $^1$H-NMR-spectrum was determined with a Perkin-Elmer R-24A apparatus using an internal tetramethylsilane standard and $CDCl_3$ as solvent.

The invention is illustrated by the following examples.

Example 1

1-Isopropylamino-3-[4-(1-acetoxy-2-methoxyethyl)phenoxy]-2-propanol

11,9 g 1,2-epoxy-3-[4-(1-acetoxy-2-methoxyethyl)phenoxy]propane and 30 ml isopropylamine are heated in an autoclave at 100°C for 1 hour or refluxed in 30 ml toluene for 4 hours. The reaction mixture is evaporated to dryness in vacuum. 14,3 g of light yellow oil is obtained, which can be used in the following example without purification.

Example 2

1-Isopropylamino-3-[4-(2-methoxyethyl)phenoxy]-2-propanol

10,5 g of the product obtained in example 1 is dissolved in 50 ml glacial acetic acid and to the solution is added 0,5 g 10% Pd/C-catalyst. The mixture is hydrogenated at 50°C at normal pressure until the calculated amount of hydrogen has been consumed. The catalyst is filtered off and the solvent evaporated in vacuum. The residue is dissolved in 150 ml water and the solution is made alkaline with $Na_2Ca_3$. The product is extracted into methylene chloride, which is washed with water and dried. The solvent is distilled off to give 7,4 g (86%) of solid metoprolol, which can be crystallized from e.g. heptane. The product having m.p of 44—45°C can be converted to the tartrate in acetone containing tartaric acid. The melting point of metoprolol tartrate is about 120°C.

Example 3

1-Isopropylamino-3-[4-(1-acetoxy-2-methoxyethyl)phenoxy]-2-propanol

10,0 g 1,2-epoxy-3-[4-(1-acetoxy-2-methoxyethyl)phenoxy]propane and 25 ml isopropylamine are heated in an autoclave at 100°C for 1 hour. After this reaction mixture is evaporated in vacuum to dryness. The residue is dissolved in 50 ml 5% acetic acid. The neutral impurities are washed into toluene. To the aqueous solution is added sodium hydrogencarbonate until the pH is about 7,5. The product is extracted with 2 x 30 ml methylene chloride and the methylene chloride is evaporated. 10,5 g almost colourless oil is obtained.

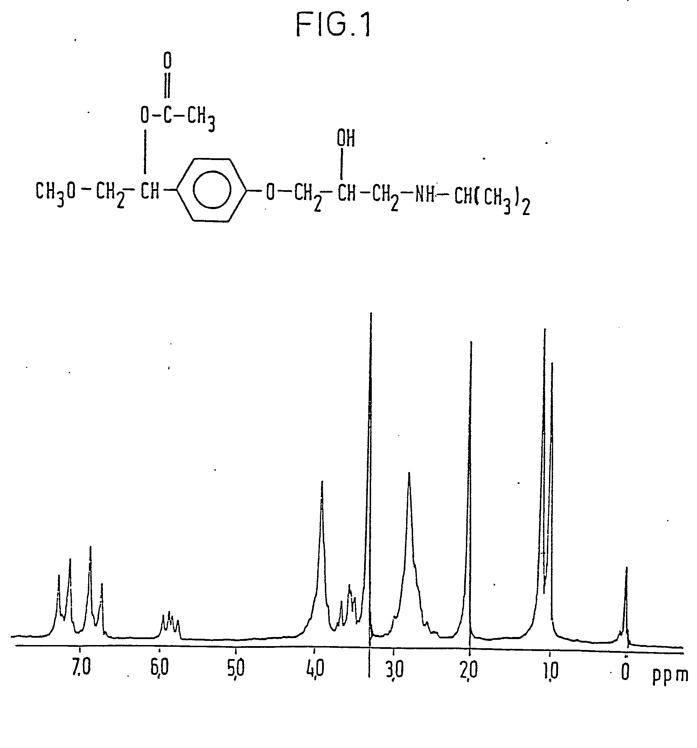

The $^1$H-NMR-spectrum of the product is shown in FIG. 1.

Example 4

1-Isopropylamino-3-[4-(2-methoxyethyl)phenoxy]-2-propanol

10,5 of the product obtained in example 3 is dissolved in 50 ml glacial acetic acid and to the solution is added 0,3 g 10% Pd/C-catalyst. The mixture is hydrogenated at 50—60°C at normal pressure until the calculated amount of hydrogen has been consumed. The catalyst is filtered off and the solvent evaporated in vacuum. The residue is dissolved in 80 ml water and washed with 1 × 20 ml toluene. The aqueous solution is made alkaline with $Na_2CO_3$ and the product extracted with toluene, washed with water and the solvent is distilled off. The residue 7,9 g (89%), crystallizes at room temperature, m.p. 45°C. The product is converted to the tartrate in acetone containing tartaric acid, m.p. of the tartrate 119—120°C.

Example 5

1-Isopropylamino-3-[4-(2-methoxyethyl)phenoxy]-2-propanol

5,0 g of 1-isopropylamino-3-[4-(1-acetoxy-2-methoxyethyl)phenoxy]-2-propanol, prepared according to example 3, is dissolved in methylene chloride. Nitrogen gas is passed into the flask and 5,0 g $NaBH_4$ is added. 20 ml trifluoroacetic acid is added dropwise at 0°C. The solution is stirred for 8 hours at this temperature and the temperature is then allowed to rise to 20—30°C. The mixture is poured into ice-water and made alkaline with sodium hydroxide. The product is extracted with methylene chloride and the solvent is distilled off. The residue is dissolved in 30 ml acetone and the pH of the solution is adjusted to 5—6 with tartaric acid. The product crystallizes at tartrate. Yield 4,2 g, m.p. 117—120°C.

Example 6

1-Isopropylamino-3-[4-(2-methoxyethyl)phenoxy]-2-propanol

The procedure of the preceding example is followed except that in place of trifluoroacetic acid is used 20 ml methanesulfonic acid. The yield of metoprolol tartrate is 4,0 g, m.p. 117—120°C.

**Claims for the Contracting States: CH IT LI NL SE**

1. A process for the preparation of the therapeutically active 1-isopropylamino-3-[4-(2-methoxyethyl)phenoxy]-2-propanol of the formula

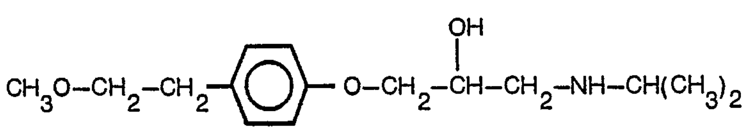

(I)

and acid addition salts thereof, characterized by

a) reacting 1,2-epoxy-3[4-(1-acetoxy-2-methoxyethyl)phenoxy]propane with isopropylamine either without solvent or in an organic solvent at elevated temperature, whereby 1-isopropylamino-3-[4-(1-acetoxy-2-methoxyethyl)phenoxy]-2-propanol of the formula

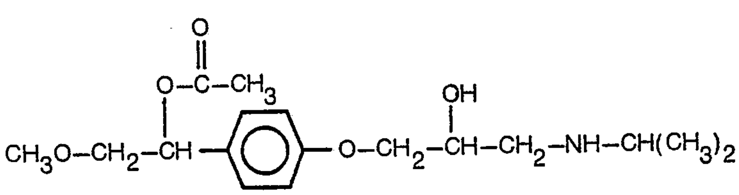

is formed which

b) is reduced to 1-isopropylamino-3-[4-(2-methoxyethyl)phenoxy]-2-propanol (I), which by conventional methods can be converted to acid addition salts.

2. A process as claimed in Claim 1, characterized thereby, that the reduction in step b) is a catalytic hydrogenation.

3. A process as claimed in Claim 1, characterized thereby, that the reduction in step b) is performed with sodium borohydride in an inert solvent containing trifluoroacetic acid or methanesulfonic acid.

4. An intermediate formed in the preparation of the therapeutically active 1-isopropylamino-3-[4-(2-methoxyethyl)phenoxy]-2-propanol, characterized by being 1-isopropylamino-3-[4-(1-acetoxy-2-methoxyethyl)phenoxy]-2-propanol of the formula

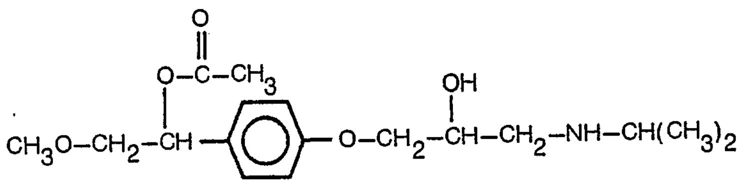

**Claims for the Contracting State: AT**

1. A process for the preparation of the therapeutically active 1-isopropylamino-3-[4-(2-methoxyethyl)phenoxy]-2-propanol of the formula

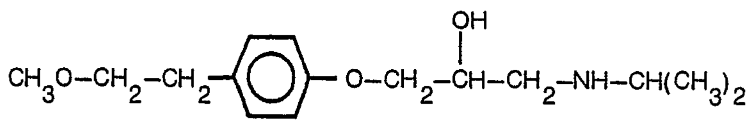

(I)

and acid addition salts thereof, characterized by

a) reacting 1,2-epoxy-3-[4-(1-acetoxy-2-methoxyethyl)phenoxy]propane with isopropylamine either without solvent or in an organic solvent at elevated temperature, whereby is formed 1-isopropylamino-3-[4-(1-acetoxy-2-methoxyethyl)phenoxy]-2-propanol of the formula

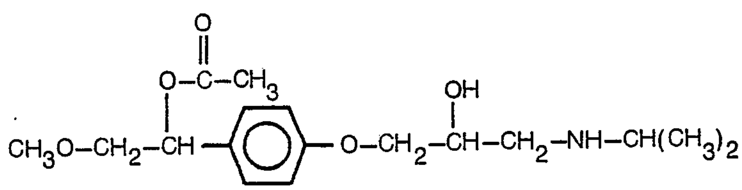

which

b) is reduced to 1-isopropylamino-3-[4-(2-methoxyethyl)phenoxy]-2-propanol (I), which be conventional methods can be converted to acid addition salts.

2. A process as claimed in Claim 1, characterized thereby, that the reduction in step b) is a catalytic hydrogenation.

3. A process as claimed in Claim 1, characterized thereby, that the reduction in step b) is performed with sodium borohydride in an inert solvent containing trifluoroacetic acid or methanesulfonic acid.

**Revendications pour les Etats contractants: CH IT LI NL SE**

1. Procédé de préparation du 1-isopropylamino-3-[4-(2-méthoxyéthyl)phénoxy]-2-propanol thérapeutiquement actif répondant à la formule

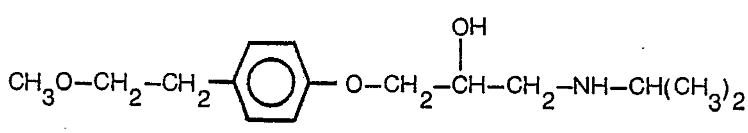

(I)

et de ses sels d'addition avec des acides, caractérisé en ce que

a) on fait réagir le 1,2-époxy-3-[4-(1-acétoxy-2-méthoxyéthyl)phénoxy]propane avec de l'isopropylamine soit sans solvant, soit dans un solvant organique à température élevée pour former du 1-isopropylamino-3-[4-(1-acétoxy-2-méthoxyéthyl)phénoxy]-2-propanol de formule

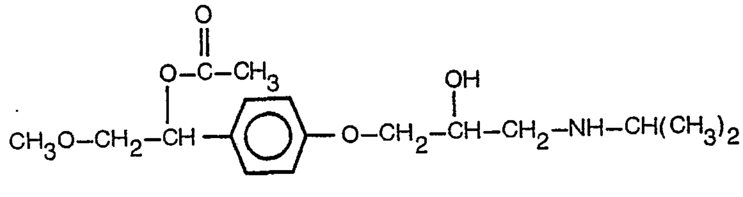

b) qui est réduit en 1-isopropylamino-3-[4-(2-méthoxyéthyl)phénoxy]-2-propanol (I) qui peut être transformé, par des méthodes classiques en des sels d'addition avec des acides.

2. Procédé selon la revendication 1, caractérisé en ce que la réduction dans l'étape (b) est une hydrogénation catalytique.

3. Procédé selon la revendication 1, caractérisé en ce que la réduction dans l'étape b) s'effectue avec du borohydrure de sodium dans un solvant inerte contenant de l'acide trifluoroacétique ou de l'acide méthanesulfonique.

4. Intermédiaire formé au cours de la préparation du 1-isopropylamino-3-[4-(2-méthoxyéthyl)-phénoxy]-2-propanol thérapeutiquement actif, caractérisé en ce qu'il est le 1-isopropylamino-3-[4-(1-acétoxy-2-méthoxyéthyl)phénoxy]-2-propanol de formule:

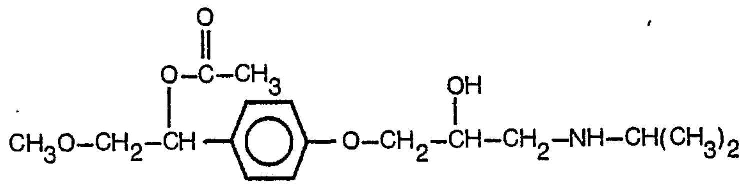

**Revendications pour l'Etat contractant: AT**

1. Procédé de préparation du 1-isopropylamino-3-[4-(2-méthoxyéthyl)phénoxy]-2-propanol thérapeutiquement actif répondant à la formule

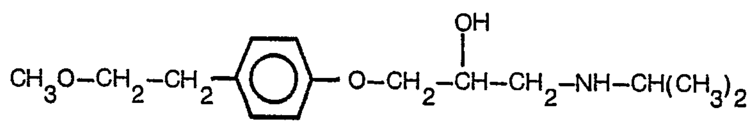

(I)

et de ses sels d'addition avec des acides, caractérisé en ce que

a) on fait réagir le 1,2-époxy-3-[4-(1-acétoxy-2-méthoxyéthyl)phénoxy]propane avec de l'isopropylamine soit sans solvant, soit dans un solvant organique à température élevée pour former du 1-isopropylamino-3-[4-(1-acétoxy-2-méthoxyéthyl)phénoxy]-2-propanol de formule

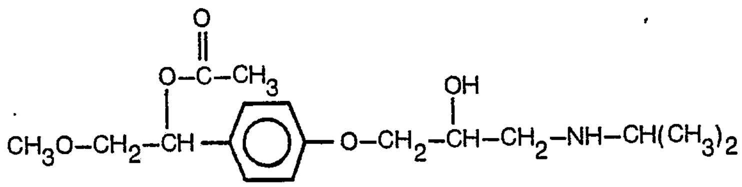

b) qui est réduit en 1-isopropylamino-3-[4-(2-méthoxyéthyl)phénoxy]-2-propanol (I) qui peut être transformé, par des méthodes classiques en des sels d'addition avec des acides.

2. Procédé selon la revendication 1, caractérisé en ce que la réduction dans l'étape (b) est une hydrogénation catalytique.

3. Procédé selon la revendication 1, caractérisé en ce que la réduction dans l'étape b) s'effectue avec du borohydrure de sodium dans un solvant inerte contenant de l'acide trifluoroacétique ou de l'acide méthanesulfonique.

**Patentansprüche für die Vertragsstraaten: CH IT LI NL SE**

1. Verfahren zur Herstellung des therapeutisch aktiven 1-Isopropylamino-3-[4-(2-methoxyäthyl)-phenoxy]-2-propanols der Formel

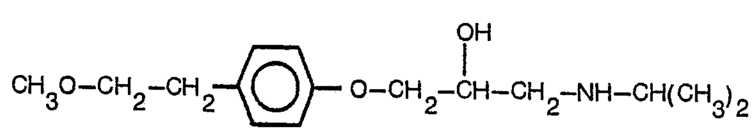

(I)

und seiner Säureadditionssalze, dadurch gekennzeichnet, daß

a) 1,2-Epoxy-3-[4-(1-acetoxy-2-methoxyäthyl)phenoxy]-propan mit Isopropylamin entweder ohne Lösungsmittel oder in einem organischen Lösungsmittel bei erhöhter Temperatur umgesetzt wird, wodurch 1-Isopropylamino-3-[4-(1-acetoxy-2-methoxyäthyl)phenoxy]-2-propanol der Formel

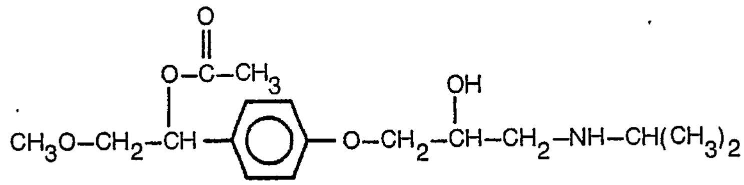

gebildet wird, welches

b) zu 1-Isopropylamino-3-[4-(2-methoxyäthyl)-phenoxy]-2-propanol (I) reduziert wird, welches mit herkömmlichen Verfahren in Säureadditionssalze umgewandelt werden kann.

2. Verfahren nach Anspruch 1, dadurch gekennzeichnet, daß die Reduktion in Schritt b) eine katalytische Hydrierung ist.

3. Verfahren nach Anspruch 1, dadurch gekennzeichnet, daß die Reduktion in Schritt b) mit Natriumborhydrid in einem inerten Lösungsmittel, welches Trifluoressigsäure oder Methansulfonsäure enthält, durchgeführt wird.

4. Zwischenprodukt, gebildet bei der Herstellung des therapeutisch aktiven 1-Isopropylamino-3-[4-(2-methoxyäthyl)-phenoxy]-2-propanols, dadurch gekennzeichnet, daß es 1-Isopropylamino-3-[4-(1-acetoxy-2-methoxyäthyl)-phenoxy]-2-propanol der Formel ist

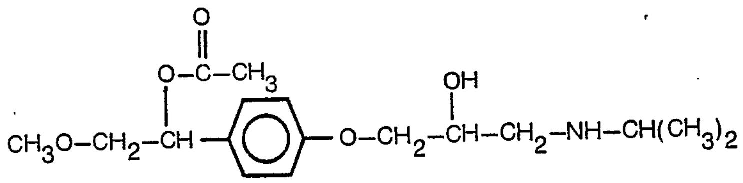

**Patentansprüche für den Vertragsstaat: AT**

1. Verfahren zur Herstellung des therapeutisch aktiven 1-Isopropylamino-3-[4-(2-methoxyäthyl)-phenoxy]-2-propanols der Formel

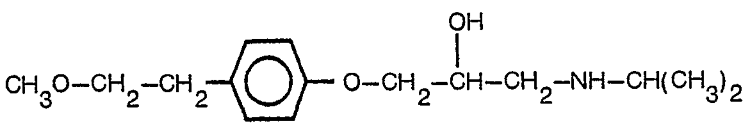

(I)

und seiner Säureadditionssalze, dadurch gekennzeichnet, daß

a) 1,2-Epoxy-3-[4-(1-acetoxy-2-methoxyäthyl)phenoxy]-propan mit Isopropylamin entweder ohne Lösungsmittel oder in einem organischen Lösungsmittel bei erhöhter Temperatur umgesetzt wird, wodurch 1-Isopropylamino-3-[4-(1-acetoxy-2-methoxyäthyl)phenoxy]-2-propanol der Formel

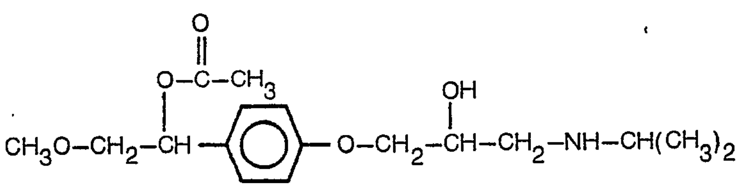

gebildet wird, welches

b) zu 1-Isopropylamino-3-[4-(2-methoxyäthyl)-phenoxy]-2-propanol (I) reduziert wird, welches mit herkömmlichen Verfahren in Säureadditionssalze umgewandelt werden kann.

2. Verfahren nach Anspruch 1, dadurch gekennzeichnet, daß die Reduktion in Schritt b) eine katalytische Hydrierung ist.

3. Verfahren nach Anspruch 1, dadurch gekennzeichnet, daß die Reduktion in Schritt b) mit Natriumborhydrid in einem inerten Lösungsmittel, welches Trifluoressigsäure oder Methansulfonsäure enthält, durchgeführt wird.

FIG.1